# EUROPEAN PATENT SPECIFICATION

(11) **EP 2 984 162 B1**
(45) Date of publication and mention of the grant of the patent: **23.10.2019**
(21) Application number: 14767876.7
(22) Date of filing: 13.03.2014
(51) Int. Cl.: C12N 1/08, C12P 23/00, C12N 9/22

(54) **USE OF THERMOPHILIC NUCLEASES FOR DEGRADING NUCLEIC ACIDS**
VERWENDUNG VON THERMOPHILEN NUKLEASEN ZUM ABBAU VON NUKLEINSÄUREN
UTILISATION DE NUCLÉASES THERMOPHILES POUR LA DÉGRADATION D'ACIDES NUCLÉIQUES

(30) Priority: 15.03.2013 US 201361794400 P; 16.01.2014 US 201461928080 P
(43) Date of publication of application: 17.02.2016
(73) Proprietor: DSM IP Assets B.V., 6411 TE Heerlen (NL)
(72) Inventor: TRUEHEART, Joshua, Somerville, MA 02142 (US); MCGRATH, Jessica, Cambridge, MA 02140 (US)
(74) Representative: Seibel-Thomsen, Nadja
(86) International application number: PCT/US2014/026379
(87) International publication number: WO 2014/151748

(56) References cited:
- WO-A1-2008/065200
- WO-A1-2009/158364
- WO-A2-2009/126890
- WO-A2-2012/135053
- US-A- 3 914 450
- US-A- 5 498 535
- US-A1- 2005 266 518
- US-A1- 2008 166 809
- US-A1- 2009 226 962
- US-A1- 2009 226 962
- US-A1- 2010 075 376
- US-A1- 2010 279 295
- BARTON E SLATKO ET AL: "Cloning, sequencing and expression of the Taq I restriction-modification system.", NUCLEIC ACIDS RESEARCH, vol. 15, no. 23, 10 December 1987 (1987-12-10), pages 9781-9796, XP055287048, GB ISSN: 0305-1048, DOI: 10.1093/nar/15.23.9781
- DATABASE UniProt [Online] 1 August 1998 (1998-08-01), Kawarabayasi,Y., et al: "SubName: Full=Uncharacterized protein {ECO:0000313|EMBL:BAA29672.1};", XP002762484, retrieved from EBI accession no. UNIPROT:O58334 Database accession no. O58334 & KAWARABAYASHI Y ET AL: "COMPLETE SEQUENCE AND GENE ORGANIZATION OF THE GENOME OF A HYPER-THERMOPHILIC ARCHAEBACTERIUM, PYROCOCCUS HORIKOSHII OT3", DNA RESEARCH, UNIVERSAL ACADEMY PRESS, JP, vol. 5, 1 January 1998 (1998-01-01), pages 55-76, XP000892259, ISSN: 1340-2838, DOI: 10.1093/DNARES/5.2.55
- Shingo Tsuji ET AL: "An Efficient Thermoinducible Bacterial Suicide System Elimination of Viable Parental Bacteria from Minicells Process Focus: production Who should read: product And process development", , 1 April 2010 (2010-04-01), XP055132320, Retrieved from the Internet: URL:http://www.bioprocessintl.com/wp-conte nt/uploads/bpi-content/BPI_A_100804AR04_O_ 90757a.pdf [retrieved on 2014-07-30]
- SLATKO ET AL.: 'Cloning, sequencing and expression of the Taq l restriction-modification system' NUCLEIC ACIDS RESEARCH vol. 15, no. 23, 1987, pages 9781 - 9796, XP055287048
- KAWARABAYASI ET AL.: '"Complete Sequence and Gene Organization of the Genome of a Hyper-thermophilic Archaebacterium, Pyrococcus horikoshii OT3' DNA RESEARCH vol. 5, 1998, pages 55 - 76, XP002948248
- Hyongi Chon ET AL: "Gene Cloning and Biochemical Characterizations of Thermostable Ribonuclease HIII from Bacillus stearothermophilus", BIOSCIENCE BIOTECHNOLOGY BIOCHEMISTRY., vol. 68, no. 10, 22 January 2004 (2004-01-22), pages 2138-2147, XP55380486, TOKYO, JAPAN ISSN: 0916-8451, DOI: 10.1271/bbb.68.2138
- COX G B ET AL: "The function of ubiquinone in Escherichia coli", BIOCHEMICAL JOURNAL, PORTLAND PRESS LTD, GB, vol. 117, no. 3, 1 April 1970 (1970-04-01) , pages 551-562, XP009109234, ISSN: 0264-6021

## Description

### FIELD OF THE INVENTION

This invention relates to a method for degrading the nucleic acids of a host cell *in vivo* and/or *in situ,* in particular when the host cell comprises a recombinant DNA, using a heterologous thermophilic nuclease. The present invention is beneficial in inactivating the biological activity of recombinant DNA in a biomass. The inactivation of the biological activity of recombinant DNA helps to prevent active recombinant DNA molecules from remaining in the end product isolated from the biomass or in the biomass itself. In addition, the inactivation of the biological activity of recombinant DNA helps to prevent active recombinant DNA molecules from being released into the environment.

### BACKGROUND OF THE INVENTION

Biotechnological production processes are increasingly employed to obtain biological compounds and fine chemicals. The progress of molecular biology techniques makes possible the mass production of a wide variety of biological compounds and fine chemicals such as proteins, antibodies, polysaccharides, antibiotics, amino acids, vitamins, alcohols, etc. Very often, in order to increase the efficiency of the production, the gene producing the desired end product(s) is genetically modified and/or introduced into a heterologous organism. The production of the desired end product(s) then takes place in fermenters controlled by modern control techniques. The end products are either the cells themselves, extracted from the cells, or collected from the cell culture broth if the compounds are found therein (either by active or passive processes).

It is common to see that the biomass produced at the end of the fermentation process contains the desired end product(s) but also active DNA molecules. Very often the active DNA molecules are recombinant DNAs. If left untreated, the recombinant DNA molecules could remain in the isolated end product and could also be released into the environment. There have been great concerns by the general public about the possible adverse effect of the remnant recombinant DNA materials in crops and food products on human health. In addition, concerns about the potential impact of the recombinant DNA on the environment has caused the authorities and institutions in most countries to issue statutory requirements and regulations calling for inactivation of the waste materials produced from the fermentation process before it being released into the environment.

There are several ways to inactivate nucleic acids. For example, nucleic acids can be inactivated physically, and most commonly, by heat. U.S. Patent No. 5,417,862 reports a method of inactivating the biological activity of DNA by heating the DNA to 60 °C to 100 °C in the presence of an acid. A similar method of degrading DNA by a combination of heat and acid is reported in U.S. Patent No. 5,118,603. The heating methods require a large amount of energy. In a large scale fermentation process, this method can be especially cost-intensive. Furthermore, depending on the nature of the end product, the harsh heat (and/or acidic) conditions could be detrimental to the integrity and activity of said product.

The nucleic acids can also be inactivated by mechanical means. U.S. Patent No. 6,165,711 reports a method using laser beams for disintegrating nucleic acids in a biologically active proteinaceous material. As another example, a method for inactivating microorganism using high-intensity pulsed polychromatic light is reported in US20020091294 A1, now abandoned. Mechanical method requires complicated light-generating devices to be used and constantly maintained and thus is cost-intensive. The light beams may disintegrate not only nucleic acids, but also other biological substances or active compounds which will cause them to lose their desired properties.

The nucleic acids can also be inactivated chemically by acids or alkali. For example, in U.S. Patent No. 7,435,567, a method using hypochlorous acids for induction of autodigestion of nucleic acids in a microorganism is reported. U.S. Patent Nos. 5,417,862 and 5,118,603 described above use other types of acid for the degradation of nucleic acids. While these methods cause the disruption of nucleic acids, the acid and alkali conditions are severe. The severe condition may cause the unwanted denaturation of certain biological compounds such as proteins. The amount of the acid and alkali used can be relatively large, which makes the method disadvantageous from the industrial production viewpoint as well.

Attempts have been made to inactive nucleic acids of an organism enzymatically, such as by using a nuclease. US20110217330 A1 reports a method for degrading host cell nucleic acids associated with vaccine production, where the method comprises a step of nucleic acid degradation by adding purified nuclease into the cell culture. In US 20040014197 A1, the construction of a transgenic bacterial strain expressing a heterologous nuclease gene in an amount effective to degrade nucleic acids is reported. While the method of adding nuclease *in vitro* such as the one reported in US20110217330 A1 causes disruption of nucleic acids, the cost is high since large amount of nuclease are required, and the efficiency of degradation is low because the cell wall of the host cell blocks the access to nucleic acids by the nuclease when added *in vitro.* The transgenic approach such as the one reported in US 20040014197 A1 allows the nuclease to be co-expressed with the host cell and thus gain access to the host nucleic acids *in vivo.* However, co-expressing a nuclease in a cell without any protection mechanism, such as methylation, will significantly stress the cell, resulting in weakened cell growth and reduced production of end product.

According to Slatko et al. (Nucleic Acids Research, vol. 15, no. 23, pp. 9781-9796, 1987) or US5,498,535) cloning and expression of thermophilic Taq I nuclease in E. coli is known.

In vivo degradation of nucleic acids is described in WO2009158364 A1, WO2008065200 A1 or by Hyongi Chon et al. (Bioscience Biotechnology Biochemistry, Vol. 68, no. 10, pp.2138-2147, 2004), the thermophilic enzymes are under the control of an inducible promoter.

Production of carotenoids or retinolic products in general using oleaginous fungi such as e.g. Yarrowia lipolytica is described in WO2009126890 A2.

Kawarabayashi et al. (DNA Research, Universal Academy Press, vol. 5, pp. 55-76, 1998) disclose the coding sequence of thermophilic nuclease Pho I.

Archaeal thermophilic nucleases are disclosed in WO2012135053 A2.

The production of biological compounds which are endogenous to the host cell, e.g. production of ubiquinone in E. coli, is described in Cox et al. (Biochemical Journal, Portland Press Ltd, GB, vol. 117, no. 3, pp. 551-562, 1970).

A method is described in US2008166809 A1 with recombinant expression of Taq I using fungal cells with the aim to improve genetic recombination frequency in genomic DNA.

The problem underlying the present invention is therefore to provide a cost-saving way to degrade the nucleic acids of a host cell that produces biological compounds and fine chemicals, especially on an industrial production scale. A further problem underlying the present invention is to provide a method in which the nucleic acids degradation process is controllable and does not impede the production of the desired end product(s) in the host cell. The above problems are solved according to the invention by the subject matter of the present claims.

### SUMMARY OF THE INVENTION

We have now surprisingly found that thermophilic nucleases can be used for degrading nucleic acid *in vivo* and/or *in situ* in a controlled manner. This invention relates to a novel method for degrading nucleic acids of a host organism where the host organism is modified or transformed with a heterologous thermophilic nuclease gene. Thermophilic nuclease is latent at temperatures at which the cell culture is grown normally to produce the desired product, but can be selectively activated at a higher temperature and thus degrades the nucleic acids of the host organism once activated. The activation can be triggered at any desired time point, such as at the harvest time of the cell culture when product formation is already complete. In addition to easy control, the application of this inventive process has the benefit of acting *in vivo* and/or *in situ* and thus improving the efficiency of the enzymatic reaction and avoids the drawbacks and problems of the existing physical or chemical methods. It has thus been made possible, by application of the disclosed invention, to obtain biological compounds and fine chemicals in the form of intact cells where the active nucleic acids content of the cell, especially recombinant DNAs, is degraded and inactivated.

The practice of this invention is broadly applicable to both microorganisms and higher eukaryotic cell cultures, and particularly industrial strains used in a large scale production of commercial end products.

The method for degrading nucleic acids *in vivo* and/or *in situ,* where the method comprises: a) introducing the gene of a thermophilic nuclease into a host cell; b) growing the host cell at a temperature at which the thermophilic nuclease is latent; and c) degrading the nucleic acids of the host cell by changing the temperature in step (b) to a temperature at which the thermophilic nuclease is active.

The present invention also provides a process for the production of biomass by fermenting a genetically modified cell being capable of producing a biological compound and which comprises a heterologous nucleic acid sequence encoding a thermophilic Taq I or Pho I nuclease gene with an enzymatic activity of 20% or less compared to the activity of said Taq I or Pho I at optimum temperature which is 65°C ± 5°C for Taq I and 75°C ± 5°C for Pho I, degrading the nucleic acids of the genetically modified cell by changing the temperature to at least 50°C and recovering the biological product isolated from the biomass; wherein the degradation is conducted during the pasteurization of the host cell , and wherein the genetically modified cell produces one or more biological products, and the biological products produced by the genetically modified cell are not the above thermophilic nuclease and are selected from the group consisting of phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin, adonixanthin, ubiquinone, vitamin K, vitamin E, retinol, retinal, retinoic acid and retinyl palmitate which is free of active nucleic acid molecules and wherein the genetically modified cell is selected from *Yarrowia, Bacillus, Escherichia, Pseudomonas, Paracoccus, Corynebacterium, Candida, Hansenula, Saccharomyces, Mortierella, Schizosaccharomyces, Aspergillus, Fusarium, Trichoderma, Crypthecodinium, Schizochytrium,* or *Thraustochytrium.* In one embodiment, the genetically modified cell is created by introducing the heterologous thermophilic nuclease gene described above into a host cell. This host cell can be either a cell which has not been genetically modified, or a cell which has been genetically modified. This host cell, before being modified with the heterologous thermophilic nuclease gene, produces one or more end products wherein said one or more end products are not the thermophilic nuclease. In another embodiment, the above mentioned host cell may be introduced first with thermophilic nuclease gene to create a genetically modified cell, and the genetically modified cell is subsequently genetically engineered to produce one or more other end products.

Another aspect of the invention relates to the use a thermophilic nuclease for degrading the nucleic acids of a host cell *in vivo* and/or *in situ,* wherein the thermophilic nuclease is heterologous to the host cell.

The temperature in step (a) of the above process invention and the method invention is optimal for the growth of the host cell. The degradation might be conducted at a temperature that is within about ± 5 °C of the optimum temperature of said thermophilic nuclease. In a specific embodiment, the degradation is conducted at the optimum temperature of said thermophilic nuclease.

In one embodiment, the DNA-degrading nuclease is TaqI nuclease. In one embodiment, the degradation is conducted at a temperature ranging between about 60 °C and about 70 °C. In a specific embodiment, the degradation is conducted at 65 °C.

In one embodiment, the host cell is selected from a group consisting of: *Yarrowia, Bacillus, Escherichia, Pseudomonas, Candida, Hansenula, Saccharomyces, Mortierella, Schizosaccharomyces, Aspergillus, Fusarium, Trichoderma, Crypthecodinium, Schizochytrium,* and *Thraustochytrium.* In a specific embodiment, the *Yarrowia* species is *Yarrowia lipolytica.*

In one embodiment, the host cell produces one or more end products selected from the group consisting of: phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin, and adonixanthin.

In one embodiment, the gene of the thermophilic nuclease is codon optimized to match the codon usage bias of the host cell. In one embodiment, the codon optimized thermophilic nuclease gene comprises the nucleic acid sequence of SEQ ID NO:1.

In a another embodiment, the invention relates to the use of TaqI nuclease for degrading recombinant DNA of a *Yarrowia lipolytica* strain *in vivo* and/or *in situ* in a process described above.

### LIST OF SEQUENCES

SEQ ID NO:1 is the DNA sequence encoding TaqI nuclease from *Thermus aquaticus,* as optimized for expression in *Yarrowia lipolytica,* and includes restriction sites for cloning and a 5' ribosomal binding site.
SEQ ID NO:2 is the non-optimized DNA sequence encoding TaqI nuclease, as isolated from *Thermus aquaticus.*
SEQ ID NO:3 is the amino acid sequence of TaqI nuclease, as deduced from SEQ ID NO:1.
SEQ ID NO:4 is the DNA sequence of the heterologous *carB* open reading frame harbored by a *Yarrowia lipolytica* strain.
SEQ ID NOs:5 and 6 are the 5' and 3' PCR primers for a 529 bp fragment within SEQ ID NO:4.
SEQ ID NO:5: primer MO4641: caatctgttgcctccctgc
SEQ ID NO:6: primer MO4642: atcctttgtgctgggacgg
SEQ ID NO:7 is the DNA sequence of the heterologous *crtZ-Dc* open reading frame harbored by a *Yarrowia lipolytica* strain.

### BRIEF DESCRIPTION OF THE FIGURES

Figures
**Fig. 1** shows the time course of DNA degradation activity in *Y. lipolytica* strains with or without the *taqI* nuclease gene.
**Fig. 2** shows DNA degradation activity at different temperatures in *Y. lipolytica* strains with or without the *taqI* nuclease gene.
**Fig. 3** shows the completeness of DNA degradation based on PCR result.
**Fig. 4** shows the production of astaxanthin and its precursors in *Y. lipolytica* strains with and without the *taqI* nuclease gene.
**Fig. 5** shows the production of zeaxanthin and its precursors in a *Y. lipolytica* strain which has the *taqI* nuclease gene.
**Fig. 6** shows the time course for degradation of DNA in *Y. lipolytica* strains producing astaxathin and zeaxanthin in fermentors.

### DETAILED DESCRIPTION OF THE INVENTION

The present invention provides a process for production of biomass as described above comprising degrading nucleic acids *in vivo* and/or *in situ* using a heterologous thermophilic nuclease selected from Taq I or Pho I as described above. The method is intended to degrade the deoxyribonucleic acids of an organism, recombinant DNA in particular, in the biological product(s) as defined herein. According to the invention, a heterologous thermophilic nuclease gene is introduced into the organism in which nucleic acids are intended to be degraded. The thermophilic nuclease is latent at the normal growth temperature of the organism, but can be activated by raising the temperature to a range around the optimum temperature of the thermophilic nuclease as defined above.

In the present invention, the thermophilic nuclease is used to inactivate the biological activity of the nucleic acids of the host organism. The term "inactivate the biological activity of nucleic acids" is considered to refer to the degradation of nucleic acids by the present invention. When nucleic acid is cleaved such that it can no longer accomplish the roles of replication, transcription or translation, it is considered to be biologically inactivated. Preferably, the nucleic acid is recombinant DNA. Preferably, the nucleic acid will be cleaved into units which are 500 base pairs or less.

In the present invention, the thermophilic nuclease is produced by the host cell rather than being added exogenously, and thus degrades the nucleic acids of the host cell *in vivo* and/or *in situ.* The term "*in vivo"* refers to the degradation of nucleic acids inside the host cell by the thermophilic nuclease produced by the cell, which may or may not be viable at the time of degradation. The term "*in situ"* refers to the degradation of nucleic acids by the thermophilic nuclease produced by the host cell after the cell wall of the host cell is broken (fully or partially). For example, an *in situ* degradation can be performed at the end of the fermentation process where cells are lysed to release their contents. Degradation can be performed immediately after the cells are lysed or after some additional processing of the lysed broth has already occurred. It is understood that degradation according to the present invention is conducted during pasteurization of the host cell.

In connection with the present invention, the term "nucleic acid" denotes single stranded, double-stranded, or partially double stranded deoxyribonucleic acid (DNA) or ribonucleic acid (RNA).

Gene: the term "gene", as used herein, generally refers to a nucleic acid encoding a polypeptide, optionally including certain regulatory elements that may affect expression of one or more gene products (i.e., RNA or protein). For example, a thermophilic nuclease gene according to the invention refers to the open reading frame (ORF) encoding a polypeptide of the thermophilic nuclease, and also to nucleic acid sequences which encode the ORF of thermophilic nuclease together with certain promoters which are placed upstream of the ORF to affect the expression of nuclease gene and/or a region for termination of transcription.

Heterologous: The term "heterologous", as used herein, refers to a gene or a polypeptide that does not naturally occur in the organism in which it is being expressed. It is understood that, where a heterologous polypeptide is to be expressed in a host cell, it will often be desirable to utilize nucleic acid sequences encoding the polypeptide that have been adjusted to accommodate codon preferences of the host cell and/or to link the encoding sequences with regulatory elements active in the host cell. For example, when the host cell is a *Yarrowia* strain (e.g., *Yarrowia lipolytica*), it will often be desirable to alter the gene sequence encoding a given polypeptide such that it conforms more closely with the codon preferences of such a *Yarrowia* strain. In certain embodiments, a gene sequence encoding a given polypeptide is altered to conform more closely with the codon preference of a species related to the host cell. Such embodiments are advantageous when the gene sequence encoding a given polypeptide is difficult to optimize to conform to the codon preference of the host cell due to experimental (e.g., cloning) and/or other reasons. In certain embodiments, the gene sequence encoding a given polypeptide is optimized even when such a gene sequence is derived from the host cell itself (and thus is not heterologous). For example, a gene sequence encoding a polypeptide of interest may be codon optimized for expression in a given host cell even though such a gene sequence is isolated from the host cell strain. In such embodiments, the gene sequence may be further optimized to account for codon preferences of the host cell. Alternately, the (non-heterologous) gene sequence might not be codon optimized but instead be linked to a regulatory element other than its own (whether that regulatory element comes from another gene in the host or from another species). Those of ordinary skill in the art will be aware of host cell codon preferences and will be able to employ the inventive methods and compositions disclosed herein to optimize expression of a given polypeptide in the host cell.

Host cell: As used herein, the "host cell" means any cell type that is susceptible to introduction of a nucleic acid construct or expression vector. The means of introduction of the nucleic acid construct or expression vector can be in the form of transformation, transfection, transduction, and the like. The term "host cell" encompasses any progeny of a parent cell that is not identical to the parent cell due to mutations that occur during replication. In preferred embodiments, the host cells produce one or more end products which are desired biological compounds or fine chemicals. It is preferred that the host cell is an industrial strain. The host cell can be a genetically modified cell or a natural cell which was non-genetically modified. A genetically modified host cell refers to a host cell which has been modified, engineered, or manipulated, often to overexpress certain biological compounds or fine chemicals.

The host cell may be any cell useful in the recombinant production of biological compounds and/or fine chemicals in a prokaryote or a eukaryote.

The term "biological compounds" is known in the art and includes compounds which are the building blocks of an organism. Examples of biological compounds include, but are not restricted to: proteins, polypeptides, amino acids, nucleic acids, nucleotides, carbohydrates, and lipids. Biological compounds according to the present invention are selected from the group consisting of phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin, adonixanthin, ubiquinone, vitamin K, vitamin E, retinol, retinal, retinoic acid and retinyl palmitate which is free of active nucleic acid molecules.

Higher animals have lost the ability to synthesize vitamins, carotenoids, cofactors and nutraceuticals and therefore need to take them in, although they are easily synthesized by other organisms such as bacteria. These molecules are either biologically active molecules per se or precursors of biologically active substances which serve as electron carriers or intermediates in a number of metabolic pathways. These compounds have, besides their nutritional value, also a significant industrial value as coloring agents, antioxidants and catalysts or other processing aids. The term "vitamin" is known in the art and includes nutrients which are required by an organism for normal functioning, but cannot be synthesized by this organism itself. The group of vitamins may include cofactors and nutraceutical compounds. The term "cofactor" includes non-protein compounds which are necessary for the occurrence of normal enzymatic activity. These compounds may be organic or inorganic; the cofactor molecules of the invention are preferably organic. The term "nutraceutical" includes food additives which promote health in organisms and animals, especially in humans. Examples of such molecules are vitamins, antioxidants and likewise certain lipids (e.g. polyunsaturated fatty acids).

Preferred fine chemicals or biosynthetic products which can be produced in organisms of the genus *Yarrowia* are carotenoids such as, for example, phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin and adonixanthin.

The prokaryotic host cell may be any gram-positive or gram-negative bacterium. Gram-positive bacteria include, but are not limited to, *Bacillus, Brevibacillus, Clostridium, Geobacillus, Lactobacillus, Lactococcus, Paenibacillus,* and *Streptomyces.* Gram-negative bacteria include, but are not limited to *E. coli* and *Pseudomonas.*

The bacterial host cell may be any *Bacillales* cell including, but not limited to, *Bacillus amyloliquefaciens, Brevibacillus brevis, Bacillus circulans, Bacillus clausii, Bacillus coagulans, Bacillus lentus, Bacillus licheniformis, Geobacillus stearothermophilus, Bacillus subtilis,* and *Bacillus thuringiensis* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The host cell may also be a eukaryote, such as a mammalian, insect, plant, algal, or fungal cell.

The host cell may be a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra*).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in *Biology and Activities of Yeast* (Skinner, F.A., Passmore, S.M., and Davenport, R.R., eds, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The algal host cell may be a *Crypthecodinium, Schizochytrium,* and *Thraustochytrium* cell such as a *Crypthecodinium cohnii, Schizochytrium sp. or Thraustochytrium sp.* cell.

The fungal host cell may be a filamentous fungal cell. "Filamentous fungi" include all filamentous forms of the subdivision Eumycota and Oomycota (as defined by Hawksworth *et al.,* 1995, *supra*)*.* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023 and Yelton et al., 1984, Proc. Natl. Acad. Sci. USA 81: 1470-1474. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163-168; and Hinnen et al., 1978, ProC. Natl. Acad. Sci. USA 75: 1929-1933.

For constructing a host cell strain with a heterologous thermophilic nuclease gene, the recombinant thermophilic nuclease gene construct is inserted into a host specific vector which allows optimal gene expression in the host. Vectors are well known in "Cloning Vectors" (Pouwels et al., Eds., Elsevier, Amsterdam-New York-Oxford, 1985). Vectors are to be understood as meaning not only plasmids, but all other vectors known to the skilled worker such as, for example, phages, viruses, such as SV40, CMV, baculovirus and adenovirus, transposons, IS elements, plasmids, cosmids, and linear or circular DNA. These vectors can be replicated autonomously in the host organism or chromosomally.

The above-described recombinant constructs according to the invention are advantageously introduced into the genome of the host organism and expressed. It is preferred to use usual cloning and transfection methods known to the skilled worker in order to bring about expression of the thermophilic nucleic acids in the expression system in question. Suitable systems are described, for example, in Current Protocols in Molecular Biology, F. Ausubel et al., Eds., Wiley Interscience, New York 1997.

The modified host cell may contain a plasmid carrying the heterologous thermophilic nuclease gene and the plasmid replicates autonomously in the host organism.

DNA is considered to be "recombinant" if it results from the application of recombinant DNA Techniques. Examples of recombinant techniques include but are not limited to cloning, mutagenesis, transformation, etc. Recombinant DNA Techniques are disclosed, for example, in Sambrook, et al., Molecular Cloning: A Laboratory Manual, Cold Spring Harbor, New York (1989) and in Ausubel FM, et al., Current Protocols in Molecular Biology, Wiley, New York (1998).

The term "nuclease" denotes an enzyme that effects hydrolytic cleavage of the ester bond between the 5'-phosphate group of a nucleotide and the 3'-hydroxy group of the adjacent nucleotide in a nucleic acid and therefore accomplishes the degradation of a DNA or RNA molecule. Nucleases are known from numerous organisms. Nucleases cleave either RNA or DNA to smaller units or even to their monomers.

In a preferred embodiment, the thermophilic nuclease according to the present invention is a DNA-degrading nuclease, and the nucleic acid which the thermophilic nuclease degrades is DNA. In a specific embodiment, the DNA being degraded is a recombinant DNA.

Although the preferred embodiment of the present invention relates to inactivation of DNA, inactivation of any nucleic acid molecule can be achieved. This includes inactivation of RNA or nucleic acid-protein conjugates such as ribozymes.

"DNA-degrading nuclease" means a nuclease which is able to cleave DNA molecules into smaller units or monomers. Such enzyme is also termed "DNase" in the state of art. "RNA-degrading nuclease" is a nuclease that is able to cleave RNA molecules into smaller units or monomers. Such enzymes are also termed "RNase" in the state of art.

The DNA nuclease can be an endonuclease or an exonuclease. An endonuclease is an enzyme that degrades a nucleic acid chain from a position inside the chain. Conversely, an exonuclease is an enzyme that degrades a nucleic acid chain from one or both ends of the chain. It is preferred for the nuclease to be an endonuclease.

"Thermophilic nuclease" means a nuclease which has its optimal activity at a temperature of 45°C or higher. Said optimal activity is observed at a temperature that is optimal for the activity of the nuclease. The optimal temperature may be determined for the nuclease in an *in vitro* assay, or in an *in vivo* assay where the nuclease is expressed in the recombinant host according to the present invention, using a suitable substrate and under assay conditions (in terms of medium composition, assay time, etc.) that are relevant for the intended application, as determined by a person skilled in the art. As an example, the temperature optimum of a nuclease such as TaqI is typically determined *in vitro* using the purified TaqI nuclease. A fixed quantity of enzyme can be incubated with one microgram of lambda DNA in a fifty microliter reaction held at a range of temperatures (typically from 25 to 85 °C) for one hour. The optimum temperature is the one at which the most digestion is observed (for example, as assayed by gel electrophoresis of the resulting lambda DNA TaqI restriction fragments). Alternately, one unit of enzyme is defined as the amount of protein required to fully digest one microgram of lambda DNA in a fifty microliter reaction in one hour at the optimum temperature. Therefore, the optimum temperature is the temperature at which the quantity of protein required to achieve full digestion is the lowest. To define the optimum temperature within the cell, a defined ratio of enzyme to DNA substrate (as used in *in vitro* assays) cannot be guaranteed. Instead, a more pragmatic approach is taken. Given that the optimum temperature *in vivo* is likely to be similar to *in vitro,* the *in vitro* defined optimum can be used as a guide. Nuclease acid digestion by the nuclease is examined at and around the *in vitro* optimal temperature. The optimal temperature is the one at which the most digestion is observed. For some nucleases, the *in vivo* optimal temperature is a range of temperatures rather than a single point, as complete digestion of genomic DNA can be observed throughout the temperature range.

Means to obtain thermophilic nucleases, i.e. Taq I or Pho I, used in the present invention include but not limited to: isolation from thermophiles, modification of homologous nuclease to acquire thermophilic activity by rational enzyme engineering and/or directed evolution. Unlike the majority of cells, thermophiles are heat-loving organisms with an optimum growth temperature of 50°C or higher. In contrast, the majority of cells grows at a lower temperature and cannot survive at the high temperature preferred by thermophiles. For example, common microbes grow at a temperature between 25 and 40 °C, mammalian cell cultures grow at around 37 °C, and plant and insect cultures grow at their respective low temperatures which were known in the art. Thermophilic nucleases lose activity quickly outside their optimum temperature and have low to no activity at the temperature at which common microbes and cell cultures grow. The levels of nuclease activity of each thermophilic nuclease at a low temperature are different and thus have to be determined individually. In the present invention, a thermophilic nuclease is considered latent at a low temperature if the nuclease activity is reduced to less than 20% of the peak activity at the optimum reaction temperature of the nuclease (either commonly known or can be determined experimentally under relevant conditions).

The best thermophilic nuclease candidates are those whose nuclease activities are low enough at the normal growth temperature so as not to impose any significant stress on the host organism, and are high enough at the optimum reaction temperature to perform efficient degradation of the nucleic acids of the host.

Owing to the property of displaying nucleic acid degradation activity at a temperature that is outside the temperature range in which a normal host organism grows, the process according to the invention can be used advantageously in degrading nucleic acids of the host cell in a controlled manner, wherein the degradation is conducted during the pasteurization of the host cell. For example, in one embodiment, the present invention discloses a process using a heterologous thermophilic nuclease gene as defined herein which is introduced into a host cell and thus is used to degrade the nucleic acid of a host cell. According to the invention, the thermophilic nuclease gene is introduced into a host cell whose nucleic acids are to be degraded. The host cell is grown at its optimum growth temperature at which the heterologous thermophilic nuclease is latent. After the host cell is grown for a period of time to a certain stage, such as at the harvest time of the cell, the activity of the thermophilic nuclease in the host call can be activated by switching the temperature to one which is optimal for the nuclease activity and as defined herein for Taq I or Pho I. By activating the thermophilic nuclease, the nucleic acids of the host cell are degraded by the nuclease selected from Taq I or Pho I, thus eliminating activities of the nucleic acids of the host cell.

One thermophilic nuclease can be introduced into a host cell and thus used to degrade the nucleic acid of a host cell. Alternatively, two or more thermophilic nuclease can be introduced into a host cell. The introduction of additional thermophilic nucleases may, for example, enhance the efficiency of the degradation of the nucleic acid.

The above method of nucleic acid degradation can be carried out at any scale ranging from lab bench to commercial scale fermentation. As described in the background section of this application, DNA of the host cell, especially recombinant DNA, is preferred to be degraded at the end of the fermentation process. The present invention provides a process for degrading the recombinant DNA in the biomass and thus inactivating any recombinant DNA. The biomass can be, for example, cells harvested from cultivation of microorganism or higher eukaryotic cell culture.

The present invention provides a fermentation process by which the recombinant DNA in the biomass is inactivated under conditions as defined herein. The methodological steps for such process are exemplified as follows: First, the gene encoding the thermophilic nuclease according to the invention is introduced into the host cell. Integration of a nucleic acid construct containing the gene of a thermophilic nuclease into the host cell can take place intrachromosomally or extrachromosomally. Second, the host cell is grown in a fermentor at a temperature which is optimal for the growth of the host cell. Third, at the end of the fermentation process, the temperature of the fermentor is raised to the optimum temperature of the thermophilic nuclease and thus activates the nuclease, wherein the degradation is conducted during the pasteurization of the host cell. The higher temperature is maintained for a period of time to allow sufficient degradation of the DNA (recombinant or not) in the host cell. Subsequently, the biomass is collected and further processed to isolate the end product. In this embodiment, the amount of end product is not affected by the action of the nuclease or the heat treatment used to activate the nuclease.

The thermophilic nuclease that is used according to the invention can be, for example, TaqI endonuclease from *Thermus aquaticus.* TaqI has an optimum temperature of 65 °C. Other thermophilic nucleases can be tested for their suitability. Examples of additional thermophilic nucleases include: Tsp509I which has an optimum temperature of 65 °C, MwoI which has an optimum temperature of 60 °C, PhoI which has an optimum temperature of 75 °C, BsaJI which has an optimum temperature of 60 °C, and BspQI which has an optimum temperature of 50°C.

Within the scope of the present invention it is preferred that the temperature for degrading nucleic acid is the temperature known to have the optimal activity of the specific thermophilic nuclease. For example, one preferred temperature for degrading nucleic acids of the host cell using thermophilic nuclease TaqI is 65 °C because TaqI is known to be most active at 65 °C. However, the optimum temperature according to the present invention is not limited to a single temperature point. According to the present invention, it is also preferred that the temperature for degrading nucleic acid is any temperature which is within a range of ±5 °C of the known optimum temperature of the thermophilic nuclease. In the case of TaqI, for example, a preferred temperature is any temperature which falls between 60 °C and 70 °C. It is particularly preferred that a temperature which falls within ±1 °C, ±2 °C, ±3 °C, or ±4 °C of the known optimum temperature of the thermophilic nuclease is the temperature for activating the thermophilic nuclease. Thus, the phrase "optimum temperature" of a thermophilic nuclease as used in the present invention refers to a temperature which is within ±5 °C of the known optimum temperature of the thermophilic nuclease.

The nucleic acid degradation process according to the present invention can be carried out relatively quickly and thus saves energy cost. The process of nucleic acid degradation might be carried out at the optimum temperature of the thermophilic nuclease for the duration of less than 48 hours, less than 24 hours, less than 1 hour, less than 40 minutes, less than 30 minutes, or less than 20 minutes. As described in the paragraph above, the optimum temperature is broadly defined as including any temperature which is within a range of ±5 °C of the known optimum temperature of the thermophilic nuclease. To the extent where the degradation of nucleic acids can be performed in a range of temperatures without jeopardizing its efficiency, a preferred temperature is the lowest temperature in the temperature range.

The present invention can be used in any host cell as defined and specified above. Examples of host cells include microorganisms and higher eukaryotic cells such as agricultural crops or animal cells in a tissue. According to the invention, the microorganisms can be for example prokaryotic cells, such as bacteria or archaebacteria, or eukaryotic cells, such as yeasts, lower or higher fungi, algae, or protozoa. According to a preferred embodiment, microorganisms are bacterial cells, fungal cells or yeast, or microalgal cells.

In certain embodiments of the invention, the organism whose nucleic acids can be degraded according to the method of the present invention is a microbe from a genus such as, but not limited to, *Yarrowia, Bacillus, Escherichia, Pseudomonas, Candida, Hansenula, Saccharomyces, Mortierella, Schizosaccharomyces, Aspergillus, Fusarium, Trichoderma, Crypthecodinium, Schizochytrium,* and *Thraustochytrium.*

In one embodiment of the invention, the host cell in accordance with the present invention is *Yarrowia lipolytica.* Advantages of *Y. lipolytica* include, for example, tractable genetics and molecular biology, availability of genomic sequence, suitability to various cost-effective growth conditions, and ability to grow to high cell density. There is already extensive commercial experience with *Y. lipolytica.*

*Escherichia coli* is useful because it is the most commonly used bacterial strain. It has been regularly used as a carrier strain for recombinant DNA.

*Saccharomyces cerevisiae* is also a useful host cell in accordance with the present invention, particularly due to its experimental tractability and the extensive experience that researchers have accumulated with the organism.

The host cell in accordance with the present invention also includes higher eukaryotic cells. The term "higher eukaryotic cell" means a eukaryotic cell of a high state of development, such as those which occur for example in animal or plant organisms. Higher eukaryotic cells do not perform all vital biochemical and metabolic functions independently and thus are grown as cell culture. The higher eukaryotic cell comprises, *inter alia,* all cells from a mammal, a plant, an insect, or an avian. According to one embodiment of the invention, the higher eukaryotic cell is from a mammal. According to another embodiment of the invention, the higher eukaryotic cell is from a plant.

According to one embodiment of the invention, the degradation of the nucleic acids of the host cell is carried out without affecting the other cellular components of the host cell. In a preferred embodiment, the degradation of the nucleic acids of the host cell is carried out without affecting the production of the biological compounds and/or fine chemicals which the host cell is engineered to produce. In one embodiment, the host cell is a *Yarrowia* strain and the biological compounds which are genetically engineered to produce include: phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin and adonixanthin. In one embodiment, the *Yarrowia* strain is a genetically modified *Yarrowia* strain. In a specific embodiment, the genetically modified *Yarrowia* strain cell introduced with a thermophilic nuclease gene as defined herein is *Yarrowia lipolytica* strain ML12924 and the biological compound produced is astaxanthin. In another embodiment, the genetically modified *Yarrowia* strain cell introduced with a thermophilic nuclease gene as defined herein is *Yarrowia lipolytica* strain ML12921 and the biological compound produced is zeaxanthin. In another embodiment, the genetically modified *Yarrowia* strain cell introduced with a thermophilic nuclease gene as defined herein is *Yarrowia lipolytica* strain ML12805 and the biological compound produced is lycopene.

In one embodiment of the invention, codon optimization is performed in order to enhance the heterologous nuclease gene in the host cell. It is known in the art that the expression of non-native genes is hampered by the existence of variation in their respective codon usage pattern compared to the host organism. To overcome these problems, codon optimization according to the present invention is performed on the thermophilic nuclease gene in order to match the host codon usage before the gene is introduced into the host cell. This process encompasses the replacement of rare codons within the DNA sequence of the nuclease gene in order to closely match the host codon usage bias while retaining 100% identity or near 100% identity to the original amino acid sequence. This process of codon optimization also allows for the simultaneous modification of predicted mRNA secondary structures that could result from changes in the GC content.

In the thermophilic nuclease of the present invention, the codon usage pattern is altered from that typical of the thermophilic nuclease gene to modify the codons without altering the coded amino acid sequence. The nucleic acid sequences for many thermophilic nuclease genes are known. In accordance with this invention, thermophilic nuclease gene segments were converted to sequences having identical translated sequences but with alternative codon usage.

In one embodiment of the invention, the codon usage pattern of the native thermophilic nuclease TaqI (SEQ ID NO:2) is optimized to the codon bias of *Y. lipolytica.* The resulting codon modified *TaqI* gene is specified in SEQ ID NO:1. The amino acid sequence encoded by SEQ ID NO:1 and SEQ ID NO:2 is specified in SEQ ID NO:3.

The codon modified *taqI* gene illustrated in SEQ ID NO:1 serves only as one example of many possible codon modified *taqI* genes. It is clear to a skilled person that any suitable codon modification methods could be used to modify the thermophilic nuclease gene according to the present invention, and the resulting sequence from using different codon modified methods may vary. Having now generally described this invention, it will become more readily understood by referencing the following specific examples which are included herein for purposes of illustration only and are not intended to be limiting unless otherwise specified.

The following examples illustrate the invention.

### EXAMPLES

**Table 1** below describes certain *Yarrowia lipolytica* strains used in the following exemplification:

**Table 1: Yarrowia lipolytica strains.**

| Strain Number | Genotype | How Constructed |
|---|---|---|
| ML8195 | *MATA erg9-4789::ura3 HMG-tr GGS carB carRP(E78G) ura3 ade1* | Classical and standard molecular genetic techniques |
| ML12805 | ML8195 [pMB6722] | Untargeted transformation |
| ML11956 | *MATB erg9-4789::URA3HMG-tr GGS carB carRP crtW crtZ* (prototrophic) | Classical and standard molecular genetic techniques |
| ML12924 | ML11956 [pMB6736] | Untargeted transformation |
| ML11218 | *MATB erg9-4789:: URA3 HMG-tr GGS carB carRP crtWcrtZ-Xa crtZ-Dc crtW-Δ6180* | Classical and standard molecular genetic techniques |
| ML12921 | ML11218 [pMB6736] | Untargeted transformation |

*Yarrowia* strains ML8195, ML12805, ML11956, ML12924, ML11218 and ML12921 were constructed by the introduction of heterologous genes under the control of the constitutive promoters (for example, *TEF1*), coupled with several generations of crossbreeding, starting with ML350 and ATCC201249 as described in U.S. Patent No. 7,851,199 B2. The *GGS* gene and the truncated *HMG* gene ("*HMG-tr*") were derived from *Yarrowia lipolytica* sequences corresponding to native geranylgeranyl pyrophosphate synthase and hydroxymethylglutaryl-CoA reductase genes, respectively. The *carRP* and *carB* genes were derived from *Mucor circinelloides,* and they encode a bifunctional phytoene synthase/lycopene cyclase and a phytoene dehydrogenase, respectively. The *crtW* gene was synthesized to encode the carotene ketolase of *Parvularcula bermudensis.* The *crtZ* gene was amplified from *Xanthobacter autotrophicus* (*Xa*), or synthesized to encode the carotene hydroxylase from *Enterobacter pulveris* (*Ep*) or *Enterobacteriaceae* bacterium DC404 (*Dc*) (SEQ ID NO:7). These genes are sometimes but not always associated with auxotrophic markers (*URA3, LEU2, URA2, LYS1, ADE1*) or a *loxP* site, remnant of a Hyg^{R} (hygromycin resistance) marker.

### Example 1: Production of plasmids for strain construction.

Plasmids were generated for expression of TaqI thermophilic nucleases as described in **Table 2.** A codon optimized *taqI* nuclease ORF sequence was synthesized *de novo* based on the sequence of the *taqI* nuclease gene of *Thermus aquaticus* (SEQ ID NO:2), using the *Y. lipolytica* codon bias as specified in SEQ ID NO:1. This codon-optimized *taqI* nuclease ORF was cleaved using NheI and MluI and ligated to pMB5082 cut with NheI and MluI to produce pMB6722. The resulting encoded TaqI protein of pMB6722 is specified in SEQ ID NO:3.

In a second plasmid, the same *taqI* nuclease gene sequence was excised from plasmid pMB6722 and cloned into a different plasmid backbone, pMB6157, to produce a new plasmid, pMB6736.

Plasmid constructions were performed based on basic molecular biology and DNA manipulation procedures. All basic molecular biology and DNA manipulation procedures described in this and other examples are generally performed according to Sambrook et al. or Ausubel et al. (Sambrook J, Fritsch EF, Maniatis T (eds). 1989. Molecular Cloning: A Laboratory Manual. Cold Spring Harbor Laboratory Press: New York; Ausubel FM, Brent R, Kingston RE, Moore DD, Seidman JG, Smith JA, Struhl K (eds). 1998. Current Protocols in Molecular Biology. Wiley: New York).

**Table 2: Plasmids**

| Plasmid | Backbone | Insert | Oligos or source |
|---|---|---|---|
| pMB6722 | pMB5082 (*URA3 tef1P-xprT*) | *taqI* | Synthesized NheI-MluI fragment |
| pMB6736 | pMB6157 (Hyg^{R} *tef1P-xprT*) | *taqI* | NheI-MluI fragment from pMB6722 |

### Example 2: Demonstration of Nuclease Activity of Y. lipolytica expressing TaqI nuclease

In this example, the gene encoding TaqI nuclease was introduced into a host strain of *Y. lipolytica.* The TaqI nuclease activity in the host strain was tested.

Plasmid pMB6722 was cleaved with Xbal and transformed into host strain ML8195 (**Table 1**). *Yarrowia* strain ML8195 is a strain which was previously genetically modified to produce lycopene. Transformants introduced with the heterologous *taqI* nuclease gene were selected on YNB glutamate plates; one such colony was selected and designated as *Yarrowia* strain ML12805. ML12805 was incubated for 3 days at 30 °C at 250 rpm in 20 mL YPD medium in a 125 mL flask. The cultures were split and 1.5 mL aliquots incubated at 65 °C or 25 °C for 5 min to 24 hr.

DNA from strains ML8195 and ML12805 after incubation at 65 °C or 25 °C was extracted and analyzed. DNA was extracted using a "Smash and Grab" yeast genomic DNA extraction protocol. Essentially, 1.5 mL of a 3-day culture was pelleted, supernatant decanted and the cells resuspended in 0.5 mL water. Cells were pelleted for 10 seconds and supernatant decanted. The pellet was resuspended in 0.2 mL Smash and Grab Solution (1% SDS, 2% Triton-X100, 100 mM NaCl, 10 mM Tris pH 8.0, 1 mM EDTA) and 0.2 mL Phenol-Chloroform-Isoamyl Alcohol, and 0.3 g glass beads were added. The tubes were vortexed for 5 min using a multi-tube holder. To the mixtures, 0.2 mL TE pH 8.0 was added and centrifuged for 5 min. The aqueous phase was transferred to a new tube and 1.0 mL cold ethanol was added and mixed to precipitate the DNA. After centrifugation for 5 min, the supernatant was removed. The pellet was resuspended in 0.4 mL TE with 75 µg/mL RNaseA and incubated at room temperature for 5 min. 10 µL 4 M ammonium acetate and 1.0 mL ethanol was added. After incubation on ice for 5 minutes and centrifugation for 10 minutes, the supernatant was discarded. The pellet was washed with 0.5 mL 70% ethanol and centrifuged 10 min. The supernatant was discarded and the pellet air dried, then resuspended in 100 µL TE. The resulting DNA from the above extraction process was analyzed by electrophoresis on 0.8% agarose gel in 1x TAE at 100 V for 1.5 h.

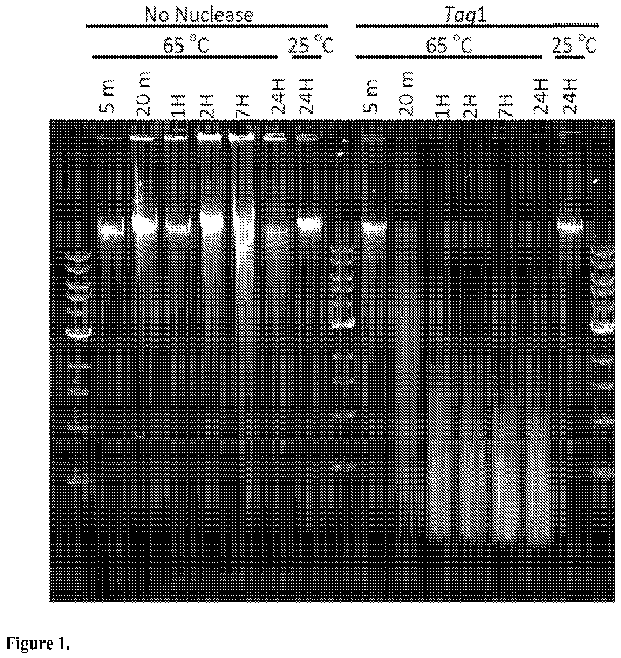

As shown in **Fig. 1****,** the host strain of *Y. lipolytica* with no *taqI* nuclease gene (ML8195) did not display DNA degradation activity at either 65 °C or 25 °C. The host strain of *Y. lipolytica* that harbors the *taqI* nuclease gene (ML12805) displayed DNA degradation activity at 65 °C but not at 25 °C. The degradation activity was observed as early as 20 minutes after the cell culture was incubated at 65 °C. The ML12805 strain did not display any DNA degradation activity at 25 °C even after being incubated for 24 hours, showing that TaqI nuclease in the *Y. lipolytica* strain is latent at 25 °C.

### Example 3: Temperature activity profile of TaqI

In this example, the activity of TaqI nuclease was examined at different temperatures.

*Y. lipolytica* strains ML12805 and ML8195 were grown for 3 days as described in Example 2. 1.5 mL aliquots of ML12805 and ML8195 cells were incubated at temperatures of 25 °C, 30 °C, 40 °C, 50 °C and 65 °C for 1.5 h and DNA was extracted and analyzed as described in Example 2.

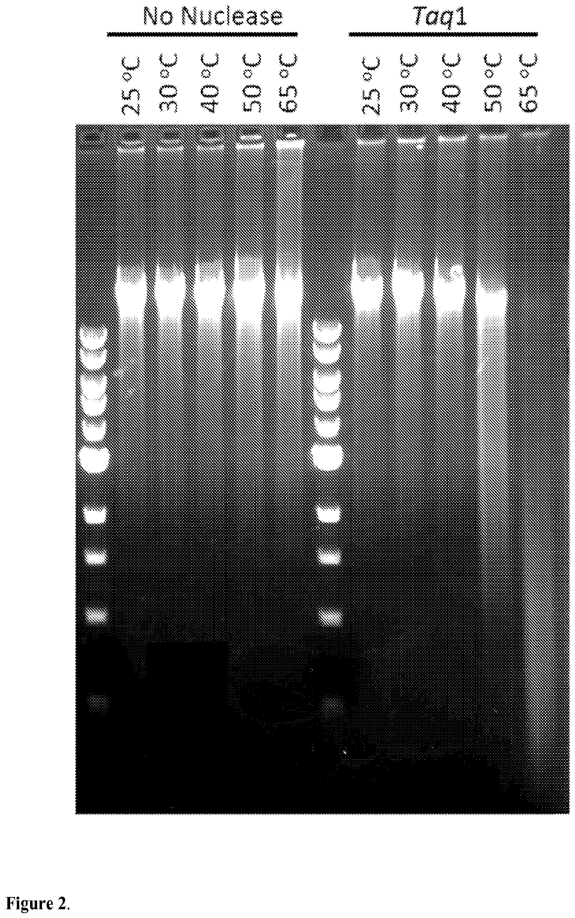

As shown in **Fig. 2****,** no DNA degradation was observed at temperatures of 25 °C, 30 °C, 40 °C, and 50 °C, in both strains ML12805 and ML8195. DNA degradation was observed at 65 °C, only in strain ML12805, as was shown in Example 2. The results show that TaqI nuclease is latent at temperatures equal to or below 50 °C.

### Example 4: PCR analysis of in vivo TaqI-digested gDNA

In this example, the degradation of the heterologous *carB* gene in a recombinant *Y. lipolytica* strain was examined.

The *carB* gene is 1740 nucleotides in length and encodes phytoene dehydrogenase, an enzyme in the carotenoid biosynthetic pathway. *carB* has been introduced into *Y. lipolytica* to enable production of carotenoids. *Y. lipolytica* strain, ML8195, a lycopene producer, harbors the *carB* gene.

The presence of the *carB* gene was examined in both strains ML8195 and ML12805 at both 25 °C and 65 °C. Serial dilutions of genomic DNA from ML12805 incubated at 25 °C or 65 °C for 24 hours were prepared. PCR was performed on the above samples using primers designed for amplifying a 529 bp fragment of the *carB* gene. The sequence of primer MO4641 is specified in SEQ ID NO:5. The sequence of primer MO4642 is specified in SEQ ID NO:6. The DNA fragment of the *carB* gene between oligos MO4641 and MO4642 contains two TaqI sites.

PCR was performed under the conditions described below. In each 25 µL reaction, 1 µL of diluted gDNA was combined with 0.5 µL of each primer (10 µM stock solution), 0.5 µL water and 22.5 µL of Platinum Taq Super Mix. The reaction parameters were 1 cycle at 94 °C for 2 min followed by 45 cycles at 94 °C for 30 s, 58 °C for 30 s and 72 °C for 60 s. A final single cycle of 72 °C for 5 min finished the PCR in a MJ Research PTC-225 Peltier Thermal Cycler. The entire reaction plus 1x loading dye was loaded onto a 2% agarose in 1xTAE gel with 0.5 µg/ml ethidium bromide and run at 100 V for 1.5 h.

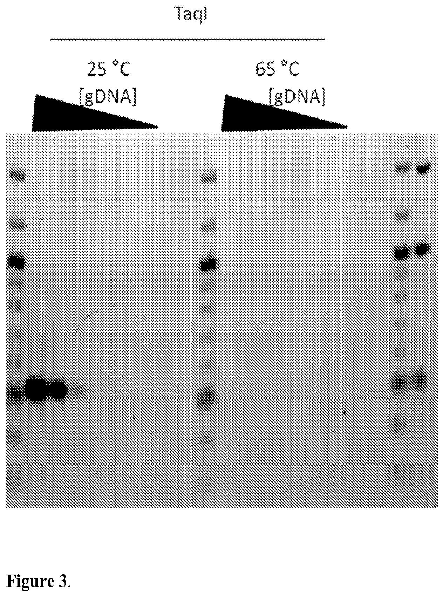

As shown in **Fig. 3****,** for the *Y. lipolytica* strain in which the *taqI* nuclease gene was introduced (ML12805), a 529 bp PCR product of *carB* was observed in the sample which was incubated at 25 °C but not in the sample which was incubated at 65 °C. These results show that the TaqI nuclease degraded the recombinant DNA (*carB* gene) in strain ML12805 when incubated at 65 °C. **Fig. 3** also illustrates that foreign DNA in strain ML12805 can be degraded to a size of less than 500 nucleotides, since no noticeable band of *carB* DNA of 529 bp was observed in the gel.

### Example 5a: Astaxanthin production in a Y. lipolytica strain harboring the taqI thermophilic nuclease gene

In this example, the production of astaxanthin in a strain of *Y. lipolytica* was examined, both before and after the *taqI* nuclease gene was introduced.

In this example, a strain of *Y. lipolytica* which produces astaxanthin (ML11956) was used. A second strain (ML12924) was constructed by introducing the *taqI* nuclease gene (MB6736) into strain ML11956. The amount of astaxanthin and its precursors in both strains was measured. Measurements were made at different time points during the cell growth period and after the host strains were incubated at 65 °C.

Strains ML11956 and ML12924 were grown in a fermentor using a fed-batch process. Samples were taken at different time points during the fermentation process and carotenoid analysis was performed according to the methods described previously in U.S. Patent No. 7,851,199 B2.

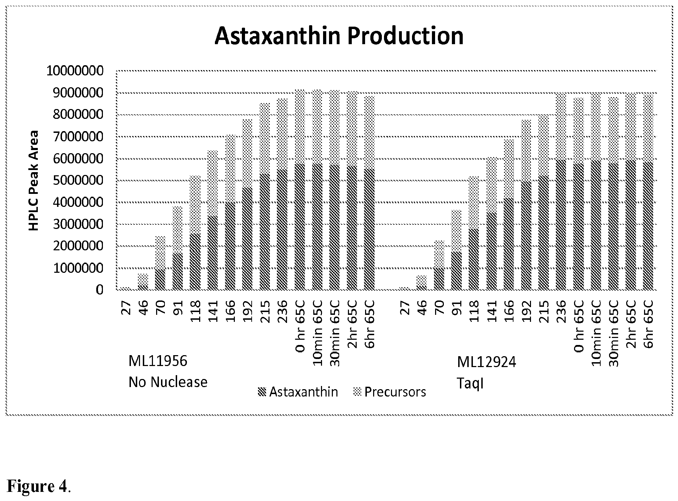

**Fig. 4** shows that the amount of astaxanthin increased as strain ML11956 grew and the production eventually reached a plateau. The amount of astaxanthin did not change after ML11956 was incubated at 65°C. In comparison, strain ML12924 behaved in the same manner as strain ML11956. The amount of astaxanthin produced by strain ML12924 was the same as in strain ML11956 throughout the fermentation period and after incubation at 65°C. The amount of astaxanthin precursors produced also remained unchanged in strains ML11956 and ML12924 before and after incubation at 65°C. This result shows that neither the introduction of the *taqI* nuclease gene nor activation of its activity affected the amount of astaxanthin produced by *Y. lipolytica.*

### Example 5b: Zeaxanthin production in a Y.lipolytica strain harboring the taqI thermophilic nuclease gene

In this example, the production of zeaxanthin in a host strain of *Y. lipolytica* with the *taqI* nuclease gene was examined.

In this example, a strain of *Y. lipolytica* which produces zeaxanthin (ML11218) was transformed with a *taqI* nuclease gene (MB6736). In the resulting *Y. lipolytica* strain (ML12921), the amount of zeaxanthin and its precursors produced were measured. Measurements were made at different time points during the cell growth period and were also made after the host strain was incubated at 65°C.

Strain ML12921 was grown in a fermentor using a fed-batch process. Samples were taken at different time points during the fermentation process and carotenoid analysis was performed according to the methods described previously in U.S. Patent No. 7,851,199 B2.

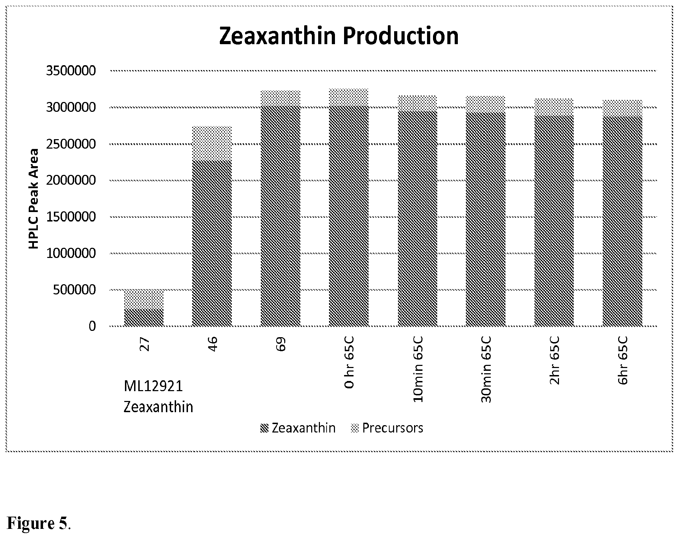

**Fig. 5** shows that the amount of zeaxanthin increased as strain ML12921 grew and the production eventually reached a plateau. The amount of zeaxanthin produced did not change after the host cells were incubated at 65°C. The amount of zeaxanthin precursors also remained the same after the host cells were incubated at 65 °C. In the control strain ML11218 (not shown), zeaxanthin production was similar to ML12921 in all the conditions tested. This result shows that activation of the TaqI nuclease activity does not affect the production of zeaxanthin in *Y. lipolytica.*

### Example 5c: Demonstration of TaqI Nuclease Activity

In this example, the TaqI nuclease activities of *Y. lipolytica* strains ML11956, ML12924, and ML12921 described in Examples 5a and 5b were examined.

Strains ML11956, ML12924, and ML12921 were grown in fermentors. For strains ML11956 and ML12924 samples were taken at 46, 121 and 236 h post inoculation. For strain ML12921 samples were taken at 46 and 69 h post inoculation. At the end of the fermentation process (236 h for astaxanthin strains and 69 h for zeaxanthin strains) the temperature was raised to 65 °C and strains were incubated at this temperature for 6 hours. Samples were taken at 0 min, 10 min, 30 min, 2 h and 6 h after the shift to the higher temperature. DNA was extracted and analyzed as described in Example 2.

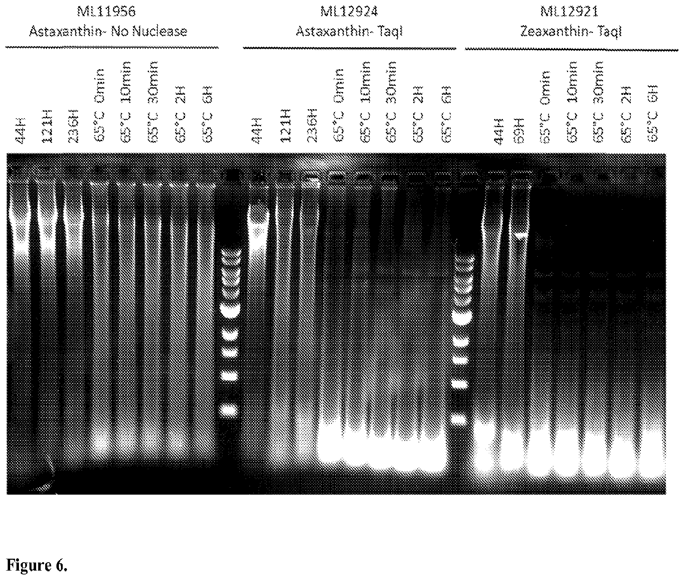

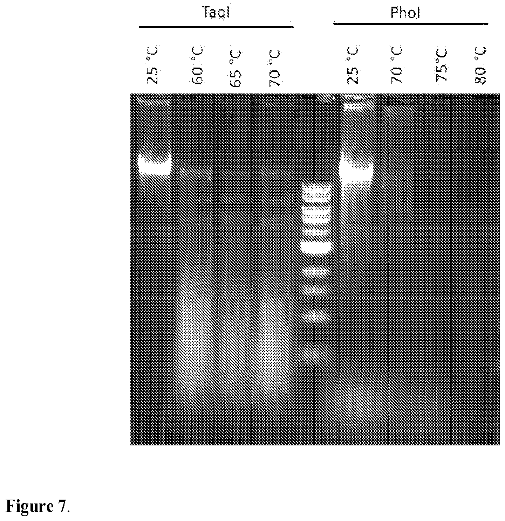

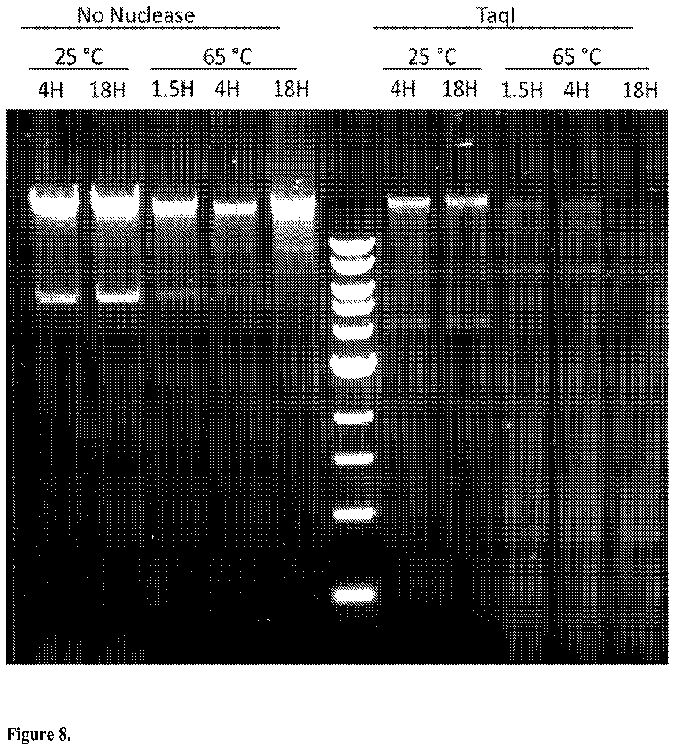

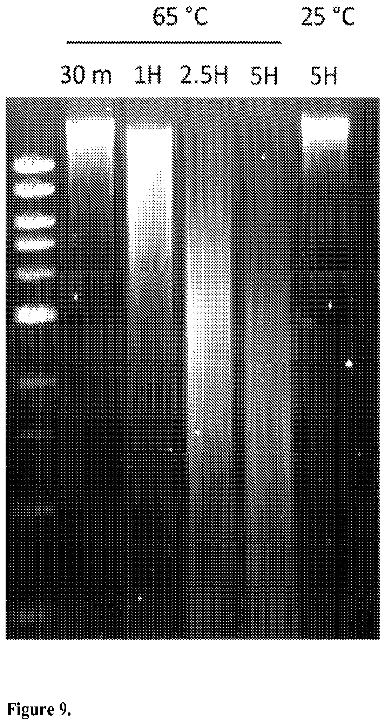

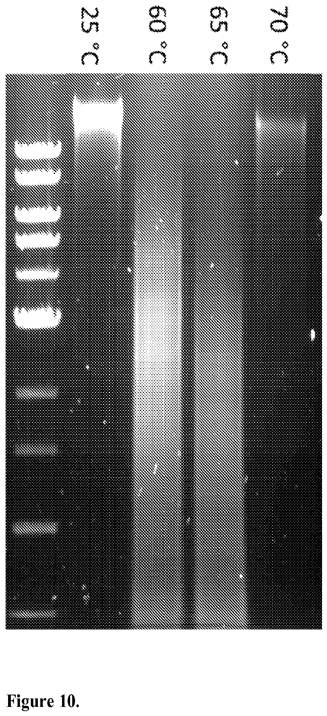

As shown in **Fig. 6****,** no DNA degradation was observed in strain ML11956. In both strains ML12924 and ML12921, DNA degradation was observed after the cells were incubated at 65 °C. The above result shows that the TaqI nuclease was latent at the fermentation temperature but was active at 65 °C in *Y. lipolytica* strains which produce astaxanthin or zeaxanthin.

All of the various aspects, embodiments, and options described herein can be combined in any and all variations.

## Claims

1. A process for the production of biomass comprising a biological compound selected from the group consisting of phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin, adonixanthin, ubiquinone, vitamin K, vitamin E, retinol, retinal, retinoic acid and retinyl palmitate which is free of active nucleic acid molecules, said process comprises:
a) fermenting a genetically modified cell at a temperature between 25°C and 40°C;
b) degrading the nucleic acids of the genetically modified cell by changing the temperature to at least 50°C; and
c) recovering the biological product isolated from the biomass;
wherein the genetically modified cell being capable of producing said biological compound and having a growth optimum between 25°C and 40°C is selected from *Yarrowia, Bacillus, Escherichia, Pseudomonas, Paracoccus, Corynebacterium, Candida, Hansenula, Saccharomyces, Mortierella, Schizosaccharomyces, Aspergillus, Fusarium, Trichoderma, Crypthecodinium, Schizochytrium,* or *Thraustochytrium,* furthermore carrying a heterologous nucleic acid sequence encoding a thermophilic Taq I or Pho I nuclease gene with an enzymatic activity of 20% or less compared to the activity of said Taq I or Pho I at optimum temperature which is 65°C ± 5°C for Taq I and 75°C ± 5°C for Pho I, and
wherein the degradation is conducted during the pasteurization of the host cell.

2. The process of claim 1, wherein the genetically modified cell is selected from *Yarrowia* or *Saccharomyces.*

3. The process of claim 2, wherein the genetically modified cell is *Yarrowia lipolytica.*

4. The process of any of claims 1 to 4, wherein the biological compound is selected from phytoene, lycopene, beta-carotene, alpha-carotene, beta-cryptoxanthin, lutein, zeaxanthin, astaxanthin, canthaxanthin, echinenone, 3-hydroxyechinenone, 3'-hydroxyechinenone, adonirubin, violaxanthin or adonixanthin.

5. The process according to any one of claims 1 to 4, wherein Taq I is encoded by a polynucleotide according to SEQ ID NO: 1 or 2 or 11, and Pho I is encoded by a polynucleotide according to SEQ ID NOs: 8, or 9.

6. The process according to any one of claims 1 to 5, wherein the nucleic acids are DNA.

7. The process according to any one of claims 1 to 6, wherein the degradation is conducted at a temperature between 60°C and 70°C in a cell carrying a heterologous Taq I gene, or wherein the degradation is conducted at a temperature between 70°C and 80°C in a cell carrying a heterologous Pho I gene.

8. The process according to claim 7, wherein the degradation is conducted at a temperature of 65°C ± 5°C in a cell carrying a heterologous Taq I gene and at a temperature of 75°C ± 5°C in a cell carrying a heterologous Pho I gene.

## Patentansprüche

1. Verfahren zur Herstellung von Biomasse, die eine biologische Verbindung ausgewählt aus der Gruppe bestehend aus Phytoen, Lycopin, Beta-Carotin, Alpha-Carotin, Beta-Cryptoxanthin, Lutein, Zeaxanthin, Astaxanthin, Canthaxanthin, Echinenon, 3-Hydroxyechinenon, 3'-Hydroxyechinenon, Adonirubin, Violaxanthin, Adonixanthin, Ubichinon, Vitamin K, Vitamin E, Retinol, Retinal, Retinsäure und Retinylpalmitat umfasst, die frei von aktiven Nukleinsäuremolekülen ist, wobei das Verfahren Folgendes umfasst:
a) Fermentieren einer gentechnisch veränderten Zelle bei einer Temperatur zwischen 25°C und 40°C;
b) Abbauen der Nukleinsäuren der gentechnisch veränderten Zelle durch Ändern der Temperatur auf wenigstens 50°C; und
c) Gewinnen des aus der Biomasse isolierten biologischen Produkts;
wobei die gentechnisch veränderte Zelle mit der Fähigkeit zur Herstellung der biologischen Verbindung und mit einem Wachstumsoptimum zwischen 25°C und 40°C ausgewählt ist aus *Yarrowia, Bacillus, Escherichia, Pseudomonas, Paracoccus, Corynebacterium, Candida, Hansenula, Saccharomyces, Mortierella, Schizosaccharomyces, Aspergillus, Fusarium, Trichoderma, Crypthecodinium, Schizochytrium* oder *Thraustochytrium,* wobei sie weiterhin eine heterologe Nukleinsäuresequenz trägt, die ein Gen für eine thermophile Taq-I- oder Pho-I-Nuklease mit einer enzymatischen Aktivität von 20% oder weniger verglichen mit der Aktivität der Taq I bzw. Pho I am Temperaturoptimum, das bei 65°C ± 5°C für Taq I und 75°C ± 5°C für Pho I liegt, codiert, und wobei der Abbau während der Pasteurisierung der Wirtszelle erfolgt.

2. Verfahren nach Anspruch 1, wobei die gentechnisch veränderte Zelle aus *Yarrowia* oder *Saccharomyces* ausgewählt ist.

3. Verfahren nach Anspruch 2, wobei es sich bei der gentechnisch veränderten Zelle um *Yarrowia lipolytica* handelt.

4. Verfahren nach einem der Ansprüche 1 bis 4, wobei die biologische Verbindung ausgewählt ist aus Phytoen, Lycopin, Beta-Carotin, Alpha-Carotin, Beta-Cryptoxanthin, Lutein, Zeaxanthin, Astaxanthin, Canthaxanthin, Echinenon, 3-Hydroxyechinenon, 3'-Hydroxyechinenon, Adonirubin, Violaxanthin oder Adonixanthin.

5. Verfahren gemäß einem der Ansprüche 1 bis 4, wobei Taq I durch ein Polynukleotid gemäß SEQ ID NO: 1, oder 2 oder 11 und Pho I durch ein Polynukleotid gemäß SEQ ID NO: 8 oder 9 codiert ist.

6. Verfahren gemäß einem der Ansprüche 1 bis 5, wobei es sich bei den Nukleinsäuren um DNA handelt.

7. Verfahren gemäß einem der Ansprüche 1 bis 6, wobei der Abbau bei einer Temperatur zwischen 60°C und 70°C in einer Zelle, die ein heterologes Taq-I-Gen trägt, erfolgt oder wobei der Abbau bei einer Temperatur zwischen 70°C und 80°C in einer Zelle, die ein heterologes Pho-I-Gen trägt, erfolgt.

8. Verfahren gemäß Anspruch 7, wobei der Abbau bei einer Temperatur von 65°C ± 5°C in einer Zelle, die ein heterologes Taq-I-Gen trägt, und bei einer Temperatur von 75°C ± 5°C in einer Zelle, die ein heterologes Pho-I-Gen trägt, erfolgt.

## Revendications

1. Processus pour la production d'une biomasse comprenant un composé biologique choisi dans le groupe constitué par les phytoène, lycopène, bêta-carotène, alpha-carotène, bêta-cryptoxanthine, lutéine, zéaxanthine, astaxanthine, canthaxanthine, échinénone, 3-hydroxy-échinénone, 3'-hydroxy-échinénone, adonirubine, violaxanthine, adonixanthine, ubiquinone, vitamine K, vitamine E, rétinol, rétinal, acide rétinoïque et palmitate de rétinyle, qui est dépourvu de molécules d'acides nucléiques actives, ledit processus comprenant les étapes consistant à :
a) faire fermenter une cellule génétiquement modifiée à une température comprise entre 25°C et 40°C ;
b) dégrader les acides nucléiques de la cellule génétiquement modifiée en changeant la température jusqu'à au moins 50°C ; et
c) récupérer le produit biologique isolé à partir de la biomasse ;
dans lequel la cellule génétiquement modifiée, étant capable de produire ledit composé biologique et ayant un optimum de croissance compris entre 25°C et 40°C, est choisie parmi *Yarrowia, Bacillus, Escherichia, Pseudomonas, Paracoccus, Corynebacterium, Candida, Hansenula, Saccharomyces, Mortierella, Schizosaccharomyces, Aspergillus, Fusarium, Trichoderma, Crypthecodinium, Schizochytrium* ou *Thraustochytrium,* transportant en plus une séquence d'acides nucléiques hétérologue qui code pour un gène de nucléase Taq I ou Pho I thermophile avec une activité enzymatique de 20% ou moins en comparaison avec l'activité desdites Taq I ou Pho I à une température optimale qui est de 65°C ± 5°C pour la Taq I et de 75°C ± 5°C pour la Pho I, et
dans lequel la dégradation est conduite pendant la pasteurisation de la cellule hôte.

2. Processus de la revendication 1, dans lequel la cellule génétiquement modifiée est choisie parmi *Yarrowia* ou *Saccharomyces.*

3. Processus de la revendication 2, dans lequel la cellule génétiquement modifiée est *Yarrowia lipolytica.*

4. Processus de l'une quelconque des revendications 1 à 4, dans lequel le composé biologique est choisi parmi le phytoène, le lycopène, le bêta-carotène, l'alpha-carotène, la bêta-cryptoxanthine, la lutéine, la zéaxanthine, l'astaxanthine, la canthaxanthine, l'échinénone, la 3-hydroxy-échinénone, la 3'-hydroxy-échinénone, l'adonirubine, la violaxanthine ou l'adonixanthine.

5. Processus selon l'une quelconque des revendications 1 à 4, dans lequel la Taq I est codée par un polynucléotide selon les SEQ ID n° : 1, 2 ou 11 et la Pho I est codée par un polynucléotide selon les SEQ ID n° : 8 ou 9.

6. Processus selon l'une quelconque des revendications 1 à 5, dans lequel les acides nucléiques sont de l'ADN.

7. Processus selon l'une quelconque des revendications 1 à 6, dans lequel la dégradation est conduite à une température comprise entre 60°C et 70°C dans une cellule transportant un gène de Taq I hétérologue ou bien dans lequel la dégradation est conduite à une température comprise entre 70°C et 80°C dans une cellule transportant un gène de Pho I hétérologue.

8. Processus selon la revendication 7, dans lequel la dégradation est conduite à une température de 65°C ± 5°C dans une cellule transportant un gène de Taq I hétérologue et à une température de 75°C ± 5°C dans une cellule transportant un gène de Pho I hétérologue.


### SEQUENCE LISTING

<110> DSM IP ASSETS B.V.
   TRUEHEART, Joshua
   MCGRATH, Jessica
<120> USE OF THERMOPHILIC NUCLEASES FOR DEGRADING NUCLEIC ACIDS
<130> 29097-WO-PCT[2]
<150> US61/794,400
   <151> 2013-03-15
<150> US61/928,080
   <151> 2014-01-16
<160> 13
<170> PatentIn version 3.5
<210> 1
   <211> 811
   <212> DNA
   <213> Artificial Sequence
<220>
   <223> Nucleotide sequence containing the codon optimized taqI gene
<400> 1
   gctagccaca aaaatggctt ccactcaggc tcagaaggct cttgagactt tcgagcgatt      60
   tctcgcttct cttgacctgg agtcctacca gcagaagtac cgacccatca agactgttga     120
   gcaggacctt ccccgagagc tgaaccccct tcccgacctg tacgagcact actggaaggc     180
   ccttgaggat aacccctcct tcctgggctt cgaggagttc tttgaccact ggtgggagaa     240
   gcgacttcga cccctcgacg agttcattcg aaagtacttt tggggttgct cctacgcctt     300
   tgttcgactt ggccttgagg ctcgactgta ccgaactgcc gtttccatct ggactcagtt     360
   tcacttctgc taccgatgga acgcctcctg cgagcttccc cttgaggccg cccccgagct     420
   tgacgcccag ggcattgacg ccctgattca cacttccggt tcctctaccg gaatccagat     480
   caagaaggag acttaccgat ccgaggccaa gtccgagaac cgatttctcc gaaagcagcg     540
   aggcaccgcc ctcatcgaga ttccctacac ccttcagact cccgaggagc ttgaggagaa     600
   ggccaagcga gcccgagtta acggagagac ttaccgactt tgggccaagg ttgctcacca     660
   ccttgaccga cttgagaacg gttttgtcat ttttcgagag tcttacgtta agtctattga      720
   gcttttcctc cagaagaacg ctcccaccct ttctggactc attcgatggg accgagttgc     780
   tcaggaggcc ctcaccgccc cctaaacgcg t                                    811
<210> 2
   <211> 792
   <212> DNA
   <213> Thermus aquaticus
<220>
   <223> taqI gene
<400> 2
   atggcttcca cacaagccca gaaagcgctc gaaacttttg agcgttttct cgcaagcttg        60
   gacctcgagt cctaccagca aaagtaccgc cctatcaaaa cggttgaaca agacctgcct       120
   agggagctga acccgcttcc ggacctgtac gagcattatt ggaaagcgct tgaggataac       180
   ccttccttcc tgggcttcga agagttcttt gaccactggt gggaaaagcg cctacggccc       240
   ttggacgagt tcatacgcaa atacttttgg ggatgctcct acgcgtttgt tcgcttgggc       300
   ctcgaggcta ggctgtaccg aacagccgtt tccatctgga ctcagtttca cttctgctac       360
   cgctggaacg cctcctgcga gcttcctcta gaagctgccc cagaactcga cgcccaaggg       420
   atagacgcgc tgattcatac aagcgggtcc tcaacaggaa tccagatcaa aaaggaaact       480
   taccgttccg aggccaagag cgagaaccgc tttttaagga agcaaagagg caccgccctc       540
   atcgagattc cctacaccct gcagacacca gaggagctcg aagaaaaagc caaacgggca       600
   agagtgaacg gagaaaccta ccgtctatgg gccaaggttg cacaccattt ggaccgtcta       660
   gaaaacggat tcgtcatttt tcgggaaagt tatgtgaaaa gcattgagct ttttctccag        720
   aaaaacgctc ctaccctatc tgggctcatc cgctgggaca gggtggccca ggaagccctc       780
   accgccccgt ga                                                           792
<210> 3
   <211> 263
   <212> PRT
   <213> Thermus aquaticus
<220>
   <223> Amino Acid sequence of TaqI nuclease
<400> 3
   Met Ala Ser Thr Gln Ala Gln Lys Ala Leu Glu Thr Phe Glu Arg Phe
   1               5                   10                  15
   Leu Ala Ser Leu Asp Leu Glu Ser Tyr Gln Gln Lys Tyr Arg Pro Ile
               20                  25                  30
   Lys Thr Val Glu Gln Asp Leu Pro Arg Glu Leu Asn Pro Leu Pro Asp
           35                  40                  45
   Leu Tyr Glu His Tyr Trp Lys Ala Leu Glu Asp Asn Pro Ser Phe Leu
       50                  55                  60
   Gly Phe Glu Glu Phe Phe Asp His Trp Trp Glu Lys Arg Leu Arg Pro
   65                  70                  75                  80
   Leu Asp Glu Phe Ile Arg Lys Tyr Phe Trp Gly Cys Ser Tyr Ala Phe
                   85                  90                  95
   Val Arg Leu Gly Leu Glu Ala Arg Leu Tyr Arg Thr Ala Val Ser Ile
               100                 105                 110
   Trp Thr Gln Phe His Phe Cys Tyr Arg Trp Asn Ala Ser Cys Glu Leu
           115                 120                 125
   Pro Leu Glu Ala Ala Pro Glu Leu Asp Ala Gln Gly Ile Asp Ala Leu
       130                 135                 140
   Ile His Thr Ser Gly Ser Ser Thr Gly Ile Gln Ile Lys Lys Glu Thr
   145                 150                 155                 160
   Tyr Arg Ser Glu Ala Lys Ser Glu Asn Arg Phe Leu Arg Lys Gln Arg
                   165                 170                 175
   Gly Thr Ala Leu Ile Glu Ile Pro Tyr Thr Leu Gln Thr Pro Glu Glu
               180                 185                 190
   Leu Glu Glu Lys Ala Lys Arg Ala Arg Val Asn Gly Glu Thr Tyr Arg
           195                 200                 205
   Leu Trp Ala Lys Val Ala His His Leu Asp Arg Leu Glu Asn Gly Phe
       210                 215                 220
   Val Ile Phe Arg Glu Ser Tyr Val Lys Ser Ile Glu Leu Phe Leu Gln
   225                 230                 235                 240
   Lys Asn Ala Pro Thr Leu Ser Gly Leu Ile Arg Trp Asp Arg Val Ala
                   245                 250                 255
   Gln Glu Ala Leu Thr Ala Pro
               260
<210> 4
   <211> 1740
   <212> DNA
   <213> Yarrowia lipolytica
<220>
   <223> carB
<400> 4
   atgtccaaga aacacattgt cattatcggt gctggcgtgg gtggcacggc tacagctgct 60
   cgtttggccc gcgaaggctt caaggtcact gtggtggaga aaaacgactt tggtggcggc 120
   cgctgctcct tgatccatca ccagggccat cgctttgatc agggcccgtc gctctacctg 180
   atgcccaagt actttgagga cgcctttgcc gatctggacg agcgcattca agaccacctg 240
   gagctgctgc gatgcgacaa caactacaag gtgcactttg acgacggtga gtcgatccag 300
   ctgtcgtctg acttgacacg catgaaggct gaattggacc gcgtggaggg cccccttggt 360
   tttggccgat tcctggattt catgaaagag acacacatcc actacgaaag cggcaccctg 420
   attgcgctca agaagaattt cgaatccatc tgggacctga ttcgcatcaa gtacgctcca 480
   gagatctttc gcttgcacct gtttggcaag atctacgacc gcgcttccaa gtacttcaag 540
   accaagaaga tgcgcatggc attcacgttt cagaccatgt atatgggcat gtcgccctac 600
   gatgcgcctg ctgtctacag cctgttgcag tacaccgagt tcgctgaagg catctggtat 660
   ccccgtggcg gcttcaacat ggtggttcag aagctagagg cgattgcaaa gcaaaagtac 720
   gatgccgagt ttatctacaa tgcgcctgtt gccaagatta acaccgatga tgccaccaaa 780
   caagtgacag gtgtaacctt ggaaaatggc cacatcatcg atgccgatgc ggttgtgtgt 840
   aacgcagatc tggtctatgc ttatcacaat ctgttgcctc cctgccgatg gacgcaaaac 900
   acactggctt ccaagaaatt gacgtcttct tccatttcct tctactggtc catgtccacc 960
   aaggtgcctc aattggacgt gcacaacatc tttttggccg aggcttatca ggagagcttt 1020
   gacgaaatct tcaaggactt tggcctgcct tctgaagcct ccttctacgt caatgtgccc 1080
   tctcgcatcg atccttctgc tgctcccgac ggcaaggact ctgtcattgt cttggtgcct 1140
   attggtcata tgaagagcaa gacgggcgat gcttccaccg agaactaccc ggccatggtg 1200
   gacaaggcac gcaagatggt gctggctgtg attgagcgtc gtctgggcat gtcgaatttc 1260
   gccgacttga ttgagcatga gcaagtcaat gatcccgctg tatggcagag caagttcaat 1320
   ctgtggagag gctcaattct gggtttgtct catgatgtgc ttcaggtgct gtggttccgt 1380
   cccagcacaa aggattctac cggtcgttat gataacctat tctttgtggg tgcaagcacg 1440
   catcccggaa ctggtgttcc cattgtcctt gcaggaagca agctcacctc tgaccaagtt 1500
   gtcaagagct ttggaaagac gcccaagcca agaaagatcg agatggagaa cacgcaagca 1560
   cctttggagg agcctgatgc tgaatcgaca ttccctgtgt ggttctggtt gcgcgctgcc 1620
   ttttgggtca tgtttatgtt cttttacttc ttccctcaat ccaatggcca aacgcccgca 1680
   tcttttatca ataatttgtt acctgaagta ttccgcgttc ataactctaa tgtcatttaa 1740
<210> 5
   <211> 19
   <212> DNA
   <213> Artificial Sequence
<220>
   <223> Primer M04641
<400> 5
   caatctgttg cctccctgc 19
<210> 6
   <211> 19
   <212> DNA
   <213> Artificial Sequence
<220>
   <223> Primer M04642
<400> 6
   atcctttgtg ctgggacgg 19
<210> 7
   <211> 558
   <212> DNA
   <213> Yarrowia lipolytica
<220>
   <223> crtZ-Dc
<400> 7
   atgcttgcgt tgtggaatac cgggatcgtg ctactgacta tcatcatcat ggaaggggtg    60
   gcaacgttcg cacacaagta catcatgcac ggctggggat ggggctggca tcattcgcac   120
   cataccccgc gcaaaggggc gtttgagcgt aacgatctct atgcggtggt gtttgcgcta   180
   ctggccattg cgctgattta cgcgggcagc gaagggtact ggccgcttca gtggattggc   240
   gcgggaatga ccggctacgg cgtgatctac tttatcgttc acgatggtct tgtccaccag   300
   cgctggccgt tccgttacgt gccgcgccgc ggctatctgc gccgcctcta catggcacac   360
   cggctgcatc acgcggtgcg ggggcgcgaa gggtgcgtct ccttcgggtt tatctacgcc   420
   ccaccggtgg acaagctgca ggcggtgctg cgcgaacgta acggcagacc cgccagcgcg   480
   ggcgctgcca gaggtgcgga tcgcgcggcg gccagctcgc cttccgggaa gccatcgcct   540
   gcttcgcgcc ggaaataa                                                 558
<210> 8
   <211> 977
   <212> DNA
   <213> Artificial Sequence
<220>
   <223> Nucleotide sequence containing the codon optimized phoI gene
<400> 8
   tgctagccac aaaaatggag atgtacaagg ttggtcgata cctcgttgat tccctccaga    60
   tttacttccc cgcttctctt gagatccagg aggagcttat taacaacggc ttctacgttc   120
   cccgatctcc tgatcgaaag gtttctatgc ccattcccat tgtttactcc gattttggtg   180
   gtcgagttat ttctattgag cgactcattc ctcccgagtg gcttgagatt tctcccgagc   240
   agctcggttg ggaggagact taccttgaga acaagcgagg tttcaagctc cctaaggagg   300
   aggtttacgt tgatgtttct atttctaacg attccattat tttcgagctt gacgttaaga   360
   actaccacct tgagcgaacc tccattcgag gcatcaaccc tgagaagtgg aagaactggg   420
   ttatgtttta cattgatctc aagtacgttg atgagttcat taacgctctt cgagagcaca   480
   tccctgcttt cgagaacaag acccgagtta ttcgagagaa gcagcagggt ggcaaggagg   540
   ttacttacta cgctaaggtt aacgtcaaga acttctctct ttgtctcggt tgtttcgatc   600
   tcgctcagcg ataccttcag atcaaggcta aggagcactg taacatttac cctggttctc   660
   ccacctgtaa cgattctctc tctaagctca agctccgact tgagtacgat ccctccatta   720
   ccacctttgc taaggttggt attgccaaga tttctggtaa gcgaccccag atcatggtta   780
   agctcacctc taccgagact aagaccattc gaggtattct caagcctgag attaagggta   840
   aggcccgagg taagctcgtt tactgtgatc accgagagaa gcgacagtac attgctcttg   900
   acctcttcga tttttacaag gccctcgttt ctactaagaa gtacgagggt aagctcccta   960
   ccgatgatta aacgcgt                                                  977
<210> 9
   <211> 957
   <212> DNA
   <213> Pyrococcus horikoshii
<220>
   <223> phoI gene
<400> 9
   atggaaatgt ataaggttgg aagatacctt gttgattccc ttcaaatata ttttcccgct      60
   tcactagaga tccaggagga gcttataaat aatggcttct acgtacccag gagcccagat     120
   agaaaagtaa gcatgccaat tccaatagta tactccgatt ttggagggag ggttatatct     180
   attgagaggt taatacctcc ggagtggctt gaaatctcgc cagagcaatt aggttgggaa     240
   gaaacttatt tagagaacaa acgtggtttt aaactaccta aagaagaggt ctacgtggat     300
   gttagtataa gcaacgattc cataatattc gagctagacg tgaaaaatta tcaccttgaa     360
   aggacatcca ttagaggcat caatcctgaa aaatggaaga attgggtgat gttttatatt     420
   gatttgaagt acgtagatga gttcattaac gctttaaggg aacatatccc agctttcgaa     480
   aataaaacaa gggttattcg tgaaaagcag caagggggca aggaagttac atattatgct     540
   aaagttaacg tcaagaattt cagcttatgc ttaggatgct tcgatctcgc tcaaaggtat     600
   cttcaaatca aagctaaaga gcactgtaac atatatccag gctcaccaac atgtaacgat     660
   tctctaagca aattaaaatt aaggcttgaa tacgatccat ccattactac ctttgctaaa     720
   gttggaatag ccaagatctc aggcaagaga ccccagatca tggttaaatt aacctctacg     780
   gaaactaaaa ctataagggg aatacttaag ccagaaataa aaggtaaggc gcgtggtaaa     840
   cttgtatatt gcgatcacag agaaaagaga cagtatatag ctttggactt gtttgatttt     900
   tacaaggcct tggtaagcac taagaagtat gaaggtaagc taccgactga tgattaa        957
<210> 10
   <211> 318
   <212> PRT
   <213> Pyrococcus horikoshii
<220>
   <223> Amino acid sequence of PhoI nuclease
<400> 10
   Met Glu Met Tyr Lys Val Gly Arg Tyr Leu Val Asp Ser Leu Gln Ile
   1               5                   10                  15
   Tyr Phe Pro Ala Ser Leu Glu Ile Gln Glu Glu Leu Ile Asn Asn Gly
               20                  25                  30
   Phe Tyr Val Pro Arg Ser Pro Asp Arg Lys Val Ser Met Pro Ile Pro
           35                  40                  45
   Ile Val Tyr Ser Asp Phe Gly Gly Arg Val Ile Ser Ile Glu Arg Leu
       50                  55                  60
   Ile Pro Pro Glu Trp Leu Glu Ile Ser Pro Glu Gln Leu Gly Trp Glu
   65                  70                  75                  80
   Glu Thr Tyr Leu Glu Asn Lys Arg Gly Phe Lys Leu Pro Lys Glu Glu
                   85                  90                  95
   Val Tyr Val Asp Val Ser Ile Ser Asn Asp Ser Ile Ile Phe Glu Leu
               100                 105                 110
   Asp Val Lys Asn Tyr His Leu Glu Arg Thr Ser Ile Arg Gly Ile Asn
           115                 120                 125
   Pro Glu Lys Trp Lys Asn Trp Val Met Phe Tyr Ile Asp Leu Lys Tyr
       130                 135                 140
   Val Asp Glu Phe Ile Asn Ala Leu Arg Glu His Ile Pro Ala Phe Glu
   145                 150                 155                 160
   Asn Lys Thr Arg Val Ile Arg Glu Lys Gln Gln Gly Gly Lys Glu Val
                   165                 170                 175
   Thr Tyr Tyr Ala Lys Val Asn Val Lys Asn Phe Ser Leu Cys Leu Gly
               180                 185                 190
   Cys Phe Asp Leu Ala Gln Arg Tyr Leu Gln Ile Lys Ala Lys Glu His
           195                 200                 205
   Cys Asn Ile Tyr Pro Gly Ser Pro Thr Cys Asn Asp Ser Leu Ser Lys
       210                 215                 220
   Leu Lys Leu Arg Leu Glu Tyr Asp Pro Ser Ile Thr Thr Phe Ala Lys
   225                 230                 235                 240
   Val Gly Ile Ala Lys Ile Ser Gly Lys Arg Pro Gln Ile Met Val Lys
                   245                 250                 255
   Leu Thr Ser Thr Glu Thr Lys Thr Ile Arg Gly Ile Leu Lys Pro Glu
               260                 265                 270
   Ile Lys Gly Lys Ala Arg Gly Lys Leu Val Tyr Cys Asp His Arg Glu
           275                 280                 285
   Lys Arg Gln Tyr Ile Ala Leu Asp Leu Phe Asp Phe Tyr Lys Ala Leu
       290                 295                 300
   Val Ser Thr Lys Lys Tyr Glu Gly Lys Leu Pro Thr Asp Asp
   305                 310                 315
<210> 11
   <211> 800
   <212> DNA
   <213> Thermus aquaticus
<220>
   <223> DNA sequence encoding TaqI nuclease
<400> 11
   ccatggcatc aacccaagca caaaaagccc tggaaacctt cgaacgcttc ctggcgagcc     60
   tggacctgga atcataccaa cagaaatacc gtccgattaa aaccgtggaa caggatctgc    120
   cgcgtgaact gaacccgctg ccggacctgt atgaacatta ctggaaagca ctggaagata    180
   atccgtcctt tctgggcttc gaagaatttt tcgatcactg gtgggaaaaa cgtctgcgcc    240
   cgctggacga atttattcgc aaatatttct ggggctgcag ctacgcgttt gttcgtctgg    300
   gtctggaagc gcgtctgtat cgcaccgccg tcagcatctg gacgcaattt catttctgct    360
   accgctggaa cgcctcttgt gaactgccgc tggaagcagc accggaactg gatgcacagg    420
   gcattgacgc tctgatccac accagtggca gctctacggg tatccaaatc aaaaaagaaa    480
   cctaccgtag tgaagcaaaa tccgaaaatc gttttctgcg caaacagcgt ggtacggctc    540
   tgattgaaat cccgtatacc ctgcaaacgc cggaagaact ggaagaaaaa gcaaaacgtg    600
   ctcgcgtcaa cggcgaaacc taccgtctgt gggcgaaagt ggcccatcac ctggatcgcc    660
   tggaaaatgg ttttgtgatt ttccgtgaat catacgttaa atcgatcgaa ctgttcctgc    720
   agaaaaacgc cccgaccctg tcgggcctga ttcgttggga ccgtgtcgca caagaagcac    780
   tgaccgctcc gtgaggtacc                                                800
<210> 12
   <211> 29
   <212> DNA
   <213> Artificial Sequence
<220>
   <223> Primer M09315
<400> 12
   cacacaccat ggcttccact caggctcag 29
<210> 13
   <211> 34
   <212> DNA
   <213> Artificial Sequence
<220>
   <223> Primer M09316
<400> 13
   cacacaggta ccttaggggg cggtgagggc ctcc 34